(19) Europäisches Patentamt
European Patent Office
Office européen des brevets

(11) Veröffentlichungsnummer: **0 138 766**
**A2**

(12) # EUROPÄISCHE PATENTANMELDUNG

(21) Anmeldenummer: **84810479.0**

(22) Anmeldetag: **01.10.84**

(51) Int. Cl.⁴: **C 07 C 13/465**, C 07 C 2/42, C 07 C 23/34, C 07 C 17/28

(30) Priorität: **07.10.83 CH 5462/83**

(43) Veröffentlichungstag der Anmeldung: **24.04.85 Patentblatt 85/17**

(84) Benannte Vertragsstaaten: **BE CH DE FR GB IT LI NL**

(71) Anmelder: **CIBA-GEIGY AG, Postfach, CH-4002 Basel (CH)**

(72) Erfinder: **Stegmann, Werner, Dr., Unter der Sonnhalde 9, CH-4410 Liestal (CH)**
Erfinder: **Meister, Daniel, Dr., Bierastrasse 17, CH-4103 Bottmingen (CH)**
Erfinder: **Widmer, Franz Rudolf, Dr., Route des Acacias 11, CH-1700 Fribourg (CH)**

(54) **Verfahren zur Herstellung von substituierten 1-Phenylindanen.**

(57) Verfahren zur Herstellung von Verbindungen der Formel I

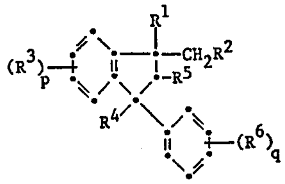

(I)

durch Dimerisierung der entsprechend α-substituierten Styrole, dadurch gekennzeichnet, dass man einen Teil des Endprodukts I als Lösungsmittel vorlegt, dass man saure Ionenaustauscher oder saure montmorillonithaltige Erden als Katalysatoren verwendet, dass man die Reaktionstemperatur über die Zudosierung des Monomeren steuert, und dass diese Temperatur im Bereich von 50° C bis 120° C liegt.

EP 0 138 766 A2

CIBA-GEIGY AG
Basel (Schweiz)

3-14608/+

Verfahren zur Herstellung von substituierten 1-Phenylindanen

Die vorliegende Erfindung betrifft ein verbessertes Verfahren zur Herstellung von 1,3,3-trisubstituierten 1-Phenylindanen aus α-substituierten Styrolverbindungen.

Es ist bekannt, dass α-methylsubstituierte Styrolverbindungen dimerisiert werden können, wobei entweder das offenkettige ungesättigte Dimere oder das gesättigte Indan-Dimere entsteht. Das gesättigte Dimere entsteht wahrscheinlich aus dem ungesättigten Dimeren, wie nachfolgend am Beispiel von α-Methylstyrol gezeigt wird:

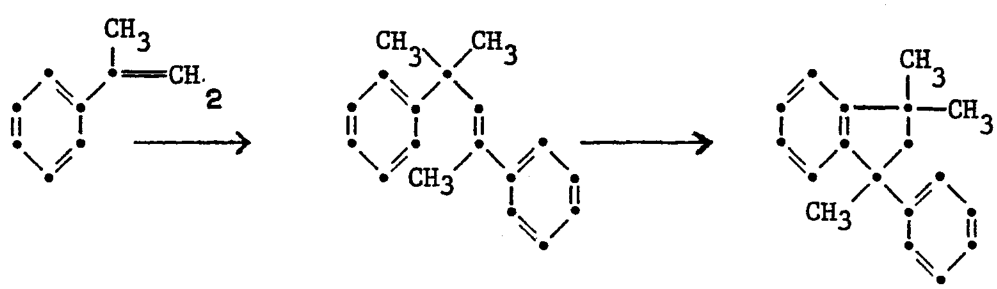

α-Methylstyrol ungesättiges Dimeres gesättigtes Dimeres

Die bisherigen Verfahren zur Herstellung der zyklischen Dimeren von α-Methylstyrolverbindungen sind durch die Anwendung bestimmter Reaktionsbedingungen gekennzeichnet, wie zum Beispiel durch Verwendung eines speziellen Katalysators, Lösungsmittels oder Autoklavs.

Als Katalysatoren sind bisher anorganische oder organische Säuren, saure Ionenaustauscher oder saure Erden verwendet worden.

Die Dimerisierungsreaktion von α-substituierten Styrolen ist sehr stark exotherm. So beträgt zum Beispiel die Wärmetönung beim α-Methylstyrol Q= -55,8 kJ/mol, was bei der Durchführung der Reaktion in der Schmelze einem adiabatischen Temperaturanstieg von ca. 220°C entspricht.

In den bisherigen Verfahren wird die Sicherheit der Prozessführung durch Verdünnen mit einem Lösungsmittel, durch eine kontinuierliche Reaktionsführung oder über die Reaktionszeiten, die Reaktionstemperaturen oder die Katalysatormengen auf Kosten von Ausbeute und Qualität erreicht.

In der DE-OS 2,659,597 wird ein Verfahren zur Herstellung von substituierten 3-Phenylindanen beschrieben, bei dem neben Schwefelsäure als Katalysator ein Anteil des Reaktionsprodukts als wärmeübertragendes Lösungsmittel vorgelegt wird, und bei dem das Monomere so zudosiert wird, dass die vorgegebene Reaktionstemperatur aufrechterhalten bleibt.

Die DE-OS 2,906,294 beschreibt ein Verfahren zur Herstellung von 1-Phenyl-1,3,3-trimethylindan durch Dimerisierung von α-Methylstyrol in inerten Lösungsmitteln in Gegenwart von Bentonit, das mit Schwefelsäure behandelt wurde, als Katalysator.

Die Dimerisierung von α-Methylstyrolen unter Verwendung von montmorillonithaltigen Erden als Katalysatoren wird in der US-PS 3,161,692 beschrieben. Die Umsetzung des Monomeren erfolgt hier bei einer Temperatur zwischen 140 und 150°C.

Es wurde nun überraschenderweise gefunden, dass man α-substituierte Styrole zu den jeweiligen 1,1,3-trisubstituierten 1-Phenylindanen bei tiefen Temperaturen in nahezu quantitativer Ausbeute dimerisieren kann, wenn spezielle saure Erden oder saure Ionenaustauscher als Katalysatoren verwendet werden, und wenn man die Prozesswärme durch den Einsatz des Reaktionsproduktes als wärmeübertragendes Lösungsmittel und über die Zudosierung des Monomeren kontrolliert. Auf diese Weise lassen sich Energiekosten einsparen und es erfolgt eine schnelle und schonende Umsetzung der Reaktanden, wodurch die Bildung von Nebenprodukten weitgehend vermieden wird, und eine im Vergleich zu bekannten Verfahren bessere Volumenausbeute erzielt wird.

Die Erfindung betrifft ein Verfahren zur Herstellung von Verbindungen der Formel I

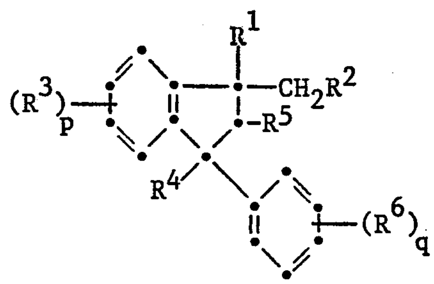 (I),

worin die beiden Reste $R^1$ und $R^4$ unabhängig voneinander $C_1$-$C_5$Alkyl, das gegebenenfalls mit ein oder mehreren Halogenatomen substituiert ist, Phenyl oder mit einer oder mehreren $C_1$-$C_5$Alkyl-, $C_1$-$C_5$Alkoxy-, Amino-, Nitro-, Cyano- oder Hydroxygruppen oder mit ein bis drei Halogenatomen substituiertes Phenyl bedeuten, worin die beiden Reste $R^2$ und $R^5$ unabhängig voneinander Wasserstoff oder $C_1$-$C_5$Alkyl sind und worin die Reste $R^3$ und $R^6$ unabhängig voneinander Wasserstoff, $C_1$-$C_5$Alkyl, Halogen oder eine Cyano-, Nitro-, Amino- oder Hydroxygruppe darstellen, und worin p die Werte 1 bis 4 und q die Werte 1 bis 5 annehmen kann, durch Dimerisation der betreffenden Monomeren der Formeln II und III

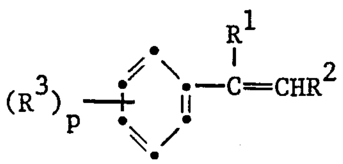 (II), 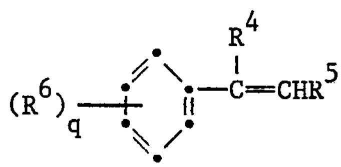 (III)

dadurch gekennzeichnet, dass man zu einer auf die jeweilige Reaktionstemperatur im Bereich von 50°C bis 120°C erwärmten Mischung aus 10 bis 100 Gew.-% des Rohprodukts der Formel I und aus 1 bis 2,5 Gew.-% saurem Ionenaustauscher oder aus 1 bis 2,5 Gew.-% saurer montmorillonithaltiger

Erde als Katalysator die Monomeren der Formeln II und III so zudosiert, dass die Temperatur des Reaktionsgemisches aufrecht erhalten bleibt; wobei die Menge des Katalysators sich auf die Summe an zugesetztem Monomeren und an vorgelegtem Rohprodukt der Formel I bezieht, und wobei die Menge an vorgelegtem Rohprodukt der Formel I sich auf die Menge an zugesetzem Monomeren bezieht.

Die Substituenten $R^1$, $R^2$, $R^3$, $R^4$, $R^5$ und $R^6$ als $C_1$-$C_5$Alkyl bedeuten beispielsweise Methyl, Ethyl, n-Propyl, n-Butyl, Isopropyl, sek.Butyl, tert.Butyl, n-Amyl oder Isoamyl.

Als halogensubstituiertes $C_1$-$C_5$Alkyl bedeuten $R^1$ und $R^4$ beispielsweise Fluor-, Chlor-, Brom- oder Iodmethyl, 1-Chlorethyl, 1,2-Dichlorethyl, 1,1-Dichlorethyl, 1,1,2-Trichlorethyl, 1-Chlorpropyl, 1-Chlorbutyl oder 1-Chlorpentyl.

Als substituiertes Phenyl bedeuten $R^1$ und $R^4$ beispielsweise o-, m- oder p-Methyl-, Ethyl-, n-Propyl-, n-Butyl, n-Pentyl-, Amino-, Nitro-, Cyano-, Hydroxy-, Methoxy, Ethoxy-, Propoxy, Butoxy- oder Pentoxyphenyl oder auch 2,3-Dimethylphenyl, 2,4-Dimethylphenyl, 2,5-Dimethylphenyl, 2,4,6-Trimethylphenyl, 2,3-Diaminophenyl, 2,4-Diaminophenyl, 2,5-Diaminophenyl, 2,6-Diaminophenyl, 2,4,6-Triaminophenyl, 2,3-Dinitrophenyl, 2,4-Dinitrophenyl, 2,5-Dinitrophenyl, 2,6-Dinitrophenyl, 2,4,6-Trinitrophenyl, 2,3-Dihydroxyphenyl, 2,4-Dihydroxyphenyl, 2,5-Dihydroxyphenyl, 2,6-Dihydroxyphenyl, 2,4,6-Trihydroxyphenyl, 2,3-Dimethoxyphenyl, 2,4-Dimethoxyphenyl, 2,5-Dimethoxyphenyl, 2,6-Dimethoxyphenyl oder 2,4,6-Trimethoxyphenyl, sowie 2-Amino-4-hydroxyphenyl, 2-Amino-4-nitrophenyl, 2-Amino-4-cyanophenyl, 2-Amino-4-methylphenyl, 2-Amino-4-methoxyphenyl, 2-Amino-4,6-dihydroxyphenyl, 2-Amino-4,6-dimethoxyphenyl, 2-Hydroxy-4-aminophenyl, 2-Hydroxy-4-nitrophenyl, 2-Hydroxy-4-nitrophenyl, 2-Hydroxy-4-cyanophenyl, 2-Hydroxy-4-methoxyphenyl, 2-Hydroxy-4-methylphenyl oder 2-Hydroxy-4,6-diaminophenyl.

$R^3$ und $R^6$ bedeuten als Halogen beispielsweise Fluor, Chlor, Brom oder Iod.

Vorzugsweise sind die beiden Substituenten $R^1$ und $R^4$ identisch. Wenn man eine Mischung von zwei oder mehreren α-substituierten Styrolverbindungen dimerisiert. wird eine Mischung von Verbindungen, die von der Formel I umfasst werden, erhalten. Wird zum Beispiel eine Mischung von α-Methylstyrol und p-Methyl-α-methylstyrol dimerisiert, so wird eine Mischung von Dimeren erhalten, worin in der Formel I $R^3$ und $R^6$ nur Wasserstoff oder nur Methyl bedeuten, oder worin $R^3$ Wasserstoff und $R^6$ Methyl bedeutet.

Das erfindungsgemässe Verfahren eignet sich zur Dimerisierung von zum Beispiel folgenden α-substituierten Styrolverbindungen: α-Methyl-p-methylstyrol, α-Ethyl-p-ethylstyrol, α-Methyl-m-isopropylstyrol, α-Methyl-p-ethylstyrol, α-Methylp-propylstyrol, α-Butyl-p-methylstyrol, α-Methyl-m-methylstyrol, α-Methyl-p-amylstyrol, 1,1-Diphenylethylen und p-Isopropenylphenol.

Beispiele für feste, saure Katalysatoren sind Ionenaustauscher auf organischer Basis, insbesondere solche mit einem Styrol-Divinyl-benzol-Copolymeren als Matrix und mit $-SO_3^-$ als Ankergruppen. Produkte dieses Typus sind beispielsweise im Handel unter der Bezeichnung "Amberlyst® 15", "Amberlite® 200" bzw. "Lewatit® SP 120" erhältlich. Diese Typen sind insbesonders zum Einsatz in nicht-wässrigen Lösungen bestimmt, zumal vor längerer Zeit festgestellt worden ist, dass saure Austauscher auch zur Katalyse in nicht-wässrigen Lösungen verwendet werden können. Es sind aber auch Ionenaustauscher vom Typ der sauren Zeolithe anwendbar.

Solche Katalysatoren sind weder in den Ausgangsprodukten noch im wasserhaltigen Reaktionsgemisch löslich. Dadurch lassen sie sich im Chargenbetrieb durch einfache Filtration abtrennen und können wiederverwendet werden.

Bevorzugt wird ein Verfahren wie oben beschrieben, bei dem Verbindungen der Formel IV durch Dimerisierung von Verbindungen der Formel II hergestellt werden

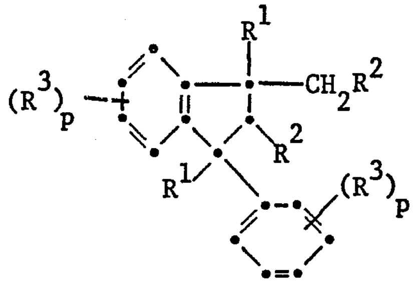

(IV).

Ebenfalls bevorzugt wird ein Verfahren zur Herstellung von Verbindungen der Formel I, bei dem Ausgangsverbindungen der Formeln II und III eingesetzt werden, worin die Substituenten $R^1$ und $R^4$ unabhängig voneinander $C_1$-$C_5$Alkyl oder Phenyl bedeuten, und worin die Substituenten $R^3$ und $R^6$ unabhängig voneinander Wasserstoff oder $C_1$-$C_5$Alkyl sind.

Besonders bevorzugt ist ein Verfahren, bei dem als Katalysator der makroporöse saure Ionenaustauscher Lewatit® SPC 118H (der Fa. Bayer) oder der saure Ionenaustauscher Nafion® H (der Fa. DuPont) verwendet wird. Um die Ausbeute zu steigern, kann es von Vorteil sein, nach Beendigung der Zudosierung das Reaktionsgemisch bei erhöhter Temperatur ausreagieren zu lassen.

Ebenfalls bevorzugt ist ein Verfahren, bei dem die saure montorillonithaltige Erde Fulcat® 22B (der Fa. Laporte, Luton, GB) als Katalysator verwendet wird, und bei dem die Reaktionstemperatur 100°C bis 110°C beträgt. Gegebenenfalls lässt man die Reaktionsmischung nach der Zudosierung der monomeren Komponente ausreagieren und arbeitet anschliessend auf.

Bevorzugt wird weiterhin ein Verfahren, bei dem als monomere Komponente der Formel II α-Methylstyrol oder 4-Chlor-α-methylstyrol eingesetzt wird.

Das erfindungsgemässe Verfahren kann in einem gewöhnlichen Reaktionsgefäss unter milden Reaktionsbedingungen, gegebenenfalls unter Inertgas durchgeführt werden. Es kann selbstverständlich auch unter Druck, das heisst, in einem geeigneten Autoklaven durchgeführt werden. Das Verfahren kann sowohl satzweise als auch halbkontinuierlich oder kontinuierlich durchgeführt werden, wobei die Zudosierung des oder der Monomeren gegebenenfalls auch automatisch erfolgen kann.

Die Reaktionsdauer beim erfindungsgemässen Verfahren ist einerseits abhängig von der eingesetzten einzelnen α-substituierten Styrolverbindung und anderenfalls von der gewählten Reaktionstemperatur, dem gegebenenfalls angewendeten Druck sowie von der Menge des verwendeten Katalysators.

Nach Beendigung der Dimerisationsreaktion kann das entstandene gesättigte Dimere mittels konventioneller Methoden isoliert und gegebenenfalls gereinigt werden. In einer bevorzugten Ausführungsform wird der Katalysator nach Beendigung der Reaktion abfiltriert und das erhaltene Produkt gegebenenfalls in einem geeigneten Lösungsmittel umkristallisiert.

Die nach dem erfindungsgemässen Verfahren erhaltenen Produkte können unter anderem als Zwischenprodukte oder als Wärmeüberträger verwendet werden. Weitere Anwendungsmöglichkeiten werden in den US-PS 3,161,692 und 3,856,752 beschrieben.

<u>Beispiel 1:</u> In einer Apparatur bestehend aus einem 750 ml Sulfierkolben mit Propellerrührer, Innenthermometer, 500 ml Tropftrichter mit Druckausgleich und Stickstoffeinleitung über Niveau mit Blasenzähler werden 142 g (0,6 Mol) rohes 1,3,3-Trimethyl-phenylindan (Gehalt 96-97 %) und 10,6 g (2,5 Gew.-% bezogen auf die Gesamtausbeute an Produkt) Fulcat® 22B vorgelegt. Das Gemisch wird unter Stickstoff auf 100°C bis 110°C aufgeheizt und ab etwa 50°C wird mit dem Rühren begonnen. Anschliessend wird das Heizbad entfernt und 283,7 g (2,4 Mol) α-Methylstyrol über einen Zeitraum von 1,5 Stunden so zudosiert, dass

die Innentemperatur des Kolbens zwischen 100°C und 110°C erhalten bleibt. Zu Beginn des zweiten Drittels der Zugabe muss wieder ein wenig geheizt werden. Nach dem Zulaufende des α-Methylstyrols wird während 30 Minuten bei einer Innentemperatur zwischen 100°C und 110°C unter Stickstoff ausgerührt. Die Endpunktsbestimmung erfolgt mittels LC. Wenn der Gehalt an Produkt ≥95 % ist, wird das Reaktionsgemisch auf 85 bis 90°C abgekühlt und anschliessend der Katalysator über eine Porzellannutsche abfiltriert. Man erhält 417 g eines blass-gelben Pulvers mit einem Gehalt an 1,1,3-Trimethyl-1-phenylindan von 96 bis 98 % entsprechend einer Ausbeute von 94,7 %. Das Produkt schmilzt bei 45 bis 48°C.

Wird eine Produktequalität von >97 % verlangt, so kristallisiert man das TPI vorzugsweise aus Methanol um (Methanol:TPI = 1,7:1). Die Gesamtausbeute liegt dann bei etwa 88 %.

Beispiel 2: In einer Apparatur bestehend aus einem 750 ml Sulfierkolben mit Propellerrührer, Innenthermometer, 500 ml Tropftrichter mit Druckausgleich und Stickstoffeinleitung über Niveau mit Blasenzähler werden 165,5 g (0,7 Mol) rohes 1,3,3-Trimethyl-1-phenylindan und 12 g Lewatit® SPC 118H (2,4 Gew.-% bezogen auf die Gesamtausbeute an Produkt) vorgelegt, und das Gemisch wird auf 50-55°C erwärmt. Innerhalb von zwei Stunden werden 328,5 g (2,78 Mol) α-Methylstyrol zudosiert. Das Reaktionsgemisch wird dann auf 150°C geheizt und während fünf Stunden bei dieser Temperatur ausgerührt. Nach dem Abfiltrieren des Katalysators werden 494 g Rohschmelze (entsprechend quantitativer Ausbeute) mit einem Gehalt an 1,3,3-Trimethyl-1-phenylindan von 93,7 % (LC) erhalten.

Beispiel 3: In einer Apparatur bestehend aus einem 750 ml Sulfierkolben mit Propellerrührer, Innenthermometer, 500 ml Tropftrichter mit Druckausgleich und Stickstoffeinleitung über Niveau mit Blasenzähler werden 118,2 g (0,5 Mol) 1,3,3-Trimethyl-1-phenylindan und 8,8 g (2,5 Gew.-% bezogen auf die Gesamtausbeute an Produkt) Nafion® H vorgelegt, und das Gemisch wird auf 100°C aufgeheizt.

Bei einer Temperatur von 100-120°C werden 234 g (1,98 Mol) α-Methylstyrol innerhalb von 15 Minuten zudosiert. Nach Beendigung des Zulaufs liegt ein Produkt mit einem Gehalt von 87 % (gemäss LC) vor. Einstündiges Ausrühren bei 150°C ergibt nach dem Abfiltrieren des Katalysators 352 g (quantitative Ausbeute) 1,3,3-Trimethyl-1-phenylindan mit einem Gehalt von 96,9 % (LC).

Beispiel 4: Ein Gemisch von 7,0 g rohes 2-(p-chlorphenyl)-propan und 1,1 g Fulcat® 22B wird in einem Kolben vorgelegt und unter Rühren bis 110°C aufgeheizt. Innerhalb 20 Minuten werden 63,0 g (0,35 Mol) 2-(p-chlorphenyl)-propan (Gehalt 85 %) zudosiert und das Gemisch wird dann 16 Stunden bei 110°C erhitzt. Das Gemisch wird gekühlt und mit 200 ml Methylenchlorid verdünnt, der Katalysator wird durch Filtration entfernt und das Lösungsmittel in vacuo destilliert. Der Rückstand wird aus 250 ml Methanol-Ethanol (4:1 v/v) mit Aktivkohle umkristallisiert. Man erhält dabei 35,1 g eines weissen Pulvers (Smp. 84-85°C, Ausbeute 59 %), dessen NMR und IR-Spektren 6-Chlor-1,3,3-trimethyl-1-(4'-chlorphenyl)-indan entsprechen.

Patentansprüche

1. Verfahren zur Herstellung von Verbindungen der Formel I

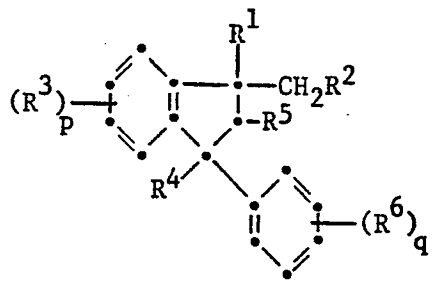

(I),

worin die beiden Reste $R^1$ und $R^4$ unabhängig voneinander $C_1$-$C_5$Alkyl, das gegebenenfalls mit ein oder mehreren Halogenatomen substituiert ist, Phenyl oder mit einer oder mehreren $C_1$-$C_5$Alkyl-, $C_1$-$C_5$Alkoxy-, Amino-, Nitro-, Cyano- oder Hydroxygruppen oder mit einem oder mehreren Halogenatomen substituiertes Phenyl bedeuten, worin die beiden Reste $R^2$ und $R^5$ unabhängig voneinander Wasserstoff oder $C_1$-$C_5$Alkyl sind und worin die Reste $R^3$ und $R^6$ unabhängig voneinander Wasserstoff, $C_1$-$C_5$Alkyl, Halogen oder eine Cyano-, Nitro-, Amino- oder Hydroxy-gruppe darstellen, und worin p die Werte 1 bis 4 und q die Werte 1 bis 5 annehmen kann, durch Dimerisation der betreffenden Monomeren der Formeln II und III

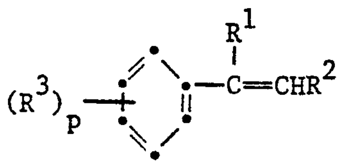

(II),

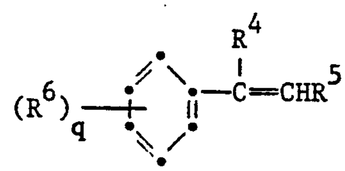

(III)

dadurch gekennzeichnet, dass man zu einer auf die jeweilige Reaktionstemperatur im Bereich von 50°C bis 120°C erwärmten Mischung aus 10 bis 100 Gew.-% des Rohprodukts der Formel I und aus 1 bis 2,5 Gew.-% saurem Ionenaustauscher oder aus 1 bis 2,5 Gew.-% saurer montmorillonithaltiger Erde als Katalysator die Monomeren der Formeln II und III so zudosiert, dass die Temperatur des Reaktionsgemisches aufrecht erhalten bleibt; wobei die Menge des Katalysators sich auf die Summe an zugesetztem Monomeren und an vorgelegtem Rohprodukt der Formel I bezieht, und wobei die Menge an vorgelegtem Rohprodukt der Formel I sich auf die Menge an zugesetztem Monomeren bezieht.

2. Verfahren gemäss Anspruch 1 zur Herstellung von Verbindungen der Formel IV, bei dem man als monomere Komponente eine Verbindung der Formel II verwendet

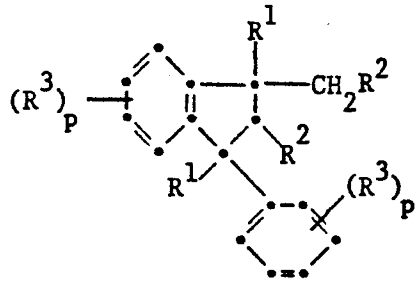

(IV).

3. Verfahren zur Herstellung von Verbindungen der Formel I gemäss Anspruch 1, bei dem Ausgansverbindungen der Formeln II und III eingesetzt werden, worin die Substituenten $R^1$ und $R^4$ unabhängig voneinander $C_1$-$C_5$Alkyl oder Phenyl bedeuten, und worin die Substituenten $R^3$ und $R^6$ unabhängig voneinander Wasserstoff oder $C_1$-$C_5$Alkyl sind.

4. Verfahren gemäss Anspruch 1, bei dem als Katalysaotr die saure montmorillonithaltige Erde Fulcat® 22B verwendet wird, und bei dem die Reaktionstemperatur 100°C bis 110°C beträgt.

5. Verfahren gemäss Anspruch 1, bei dem als Katalysator der makroporöse saure Ionenaustauscher Lewatit® SPC 118H oder der saure Ionenaustauscher Nafion® H verwendet wird.

6. Verfahren gemäss Anspruch 2, bei dem als monomere Komponente der Formel II α-Methylstyrol verwendet wird.

7. Verfahren gemäss Anspruch 2, bei dem als monomere Komponente der Formel II 4-Chlor-α-methylstyrol verwendet wird.